(19) Europäisches Patentamt
European Patent Office
Office européen des brevets

(11) EP 1 637 144 A1

(12) EUROPEAN PATENT APPLICATION
published in accordance with Art. 158(3) EPC

(43) Date of publication:
**22.03.2006 Bulletin 2006/12**

(21) Application number: **04735360.2**

(22) Date of filing: **28.05.2004**

(51) Int Cl.:
***A61K 31/7088*** *(2000.01)* ***A61K 9/127*** *(1990.01)*
***A61K 47/18*** *(1990.01)* ***A61K 47/24*** *(1990.01)*

(86) International application number:
**PCT/JP2004/007785**

(87) International publication number:
**WO 2004/105774 (09.12.2004 Gazette 2004/50)**

(84) Designated Contracting States:
**AT BE BG CH CY CZ DE DK EE ES FI FR GB GR HU IE IT LI LU MC NL PL PT RO SE SI SK TR**

(30) Priority: **30.05.2003 JP 2003154798**
**12.11.2003 JP 2003382819**
**31.03.2004 JP 2004104036**

(71) Applicant: **Nippon Shinyaku Co., Ltd.**
**Kyoto-shi,**
**Kyoto 601-8550 (JP)**

(72) Inventors:
- **YANO, Junichi**
**Nara-shi, Nara 6308105 (JP)**
- **HIRABAYASHI, K.,**
**307, Green Palace Kaikonoyashiro**
**Kyoto-shi,**
**Kyoto 6168105 (JP)**
- **YAMAGUCHI, Tohru**
**Takatsuki-shi, Osaka 569-0056 (JP)**
- **SONOKE, Satoru**
**Tsukuba-shi, Ibaraki 3050003 (JP)**

(74) Representative: **Vossius & Partner**
**Siebertstrasse 4**
**81675 München (DE)**

# (54) OLIGONUCLEIC ACID-BEARING COMPOSITE AND PHARMACEUTICAL COMPOSITION CONTAINING THE COMPOSITE

(57) The present invention provides a complex of a cationic liposome with an oligo nucleic acid, wherein the liposome comprises 2-O-(2-diethylaminoethyl)carbamoyl-1,3-O-dioleoylglycerol and a phospholipid as main ingredients. Also, the present invention provides a pharmaceutical composition containing the complex to be used for treating or preventing diseases caused by a target molecule of the oligo nucleic acid (target DNA, target RNA, target protein) since it is possible to administer the complex in vivo and to put the complex into practical use as a medicament because the complex can exhibit pharmacological efficacy in vivo.

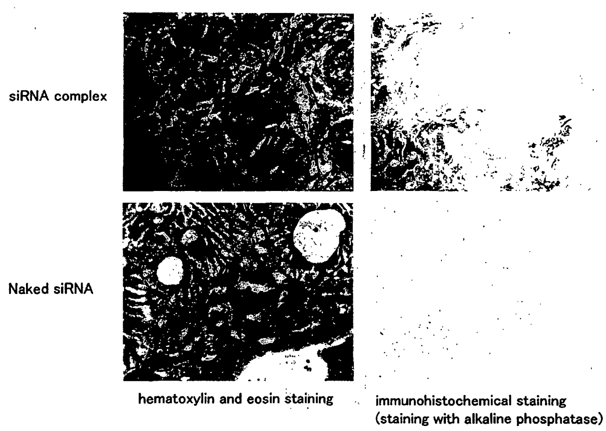


Figure 7 : Localization of siRNA complex in foci of metastasized liver tumor

EP 1 637 144 A1

SEQUENCE LISTING

<110> Nippon Shinyaku Co., LTD.

<120> oligonucleotide carrying complex and pharmaceutical composition comprising it.

<130> YCT-941

<160> 12

<210> 1
<211> 21
<212> DNA
<213> Artificial sequence

<220>
<223> synthesized DNA/RNA; sense strand of bcl-2 siRNA, wherein 2 nucleotides of 3'-terminus are constructed with DNA, and the rest are RNA.

<400> 1
gugaaguca acaugccugct t 21

<210> 2
<211> 21

<212> DNA

<213> Artificial sequence

<220>

<223> synthesized DNA/RNA; antisense strand of bcl-2 siRNA, wherein 2 nucleotides of 3'-terminus are constructed with DNA, and the rest are RNA.

<400> 2

gcaggcaugu ugacuucact t 21

<210> 3

<211> 21

<212> DNA

<213> Artificial sequence

<220>

<223> synthesized DNA/RNA; sense strand of firefly luciferase siRNA, wherein 2 nucleotides of 3'-terminus are constructed with DNA, and the rest are RNA.

<400> 3

cuuacgcuga guacuucgat t 21

<210> 4

<211> 21

<212> DNA

<213> Artificial sequence

<220>

<223> synthesized DNA/RNA; antisense strand of firefly luciferase siRNA, wherein 2 nucleotides of 3'-terminus are constructed with DNA, and the rest are RNA.

<400> 4

ucgaaguacu cagcguaagt t 21

<210> 5

<211> 18

<212> DNA

<213> Artificial sequence

<220>

<223> synthesized DNA; bcl-2 antisense DNA, wherein 1-17 nucleotides are phosphorothioate backbone modified nucleotides.

<400> 5

tctcccagcg tgcgccat 18

<210> 6

<211> 18

<212> DNA

<213> Artificial sequence

<220>

<223> synthesized DNA; reverse sequence of bcl-2 antisense DNA, wherein 1-17 position nucleotides are phosphorothioate backbone modified nucleotides.

<400> 6

taccgcgtgc gaccctct 18

<210> 7

<211> 31

<212> DNA

<213> Artificial sequence

<220>

<223> synthesized DNA; bcl-2 DNAzyme, wherein these nucleotides are phosphorothioate backbone modified nucleotides except for 8,10,12,14,16,18,20,22,23 and 31 position nucleotides.

<400> 7

ggccacaagg ctagctacaa cgacctcccc c 31

<210> 8

<211> 31

<212> DNA

<213> Artificial sequence

<220>

<223> synthesized DNA; bcl-2 DNAzyme mutant, wherein these nucleotides are phosphorothioate backbone modified nucleotides except for 8,10,12,14,16,18,20,22,23 and 31 position nucleotides.

<400> 8

ggccacaagg cgacctacaa cgacctcccc c 31

<210> 9

<211> 21

<212> DNA

<213> Artificial sequence

<220>

<223> synthesized DNA/RNA; sense strand of bcl-2 siRNA, wherein 2 nucleotides of 3'-terminus are constructed with DNA, and the rest are RNA.

<400> 9

gugaugaagu acauccauut t 21

<210> 10

<211> 21

<212> DNA

<213> Artificial sequence

<220>

<223> synthesized DNA/RNA; antisense strand of bcl-2 siRNA, wherein 2 nucleotides of 3'-terminus are constructed with DNA, and the rest are RNA.

<400> 10

aauggaugua cuucaucact t 21

<210> 11

<211> 27

<212> DNA

<213> Artificial sequence

<220>

<223> synthesized DNA/RNA; sense strand of bcl-2 siRNA, wherein 2 nucleotides of 3'-terminus are constructed with DNA, and the rest are RNA.

<400> 11

gagauaguga ugaaguacau ccauutt 27

<210> 12

<211> 27

<212> DNA

<213> Artificial sequence

<220>

<223> synthesized DNA/RNA; antisense strand of bcl-2 siRNA, wherein 2 nucleotides of 3'-terminus are constructed with DNA, and the rest are RNA.

<400> 12

aauggaugua cuucaucacu aucuctt 27

## Description

### Technical Field

The present invention relates to a complex of a cationic liposome with an oligo nucleic acid, wherein the liposome comprises 2-O-(2-diethylaminoethyl)carbamoyl-1,3-O-dioleoylglycerol and a phospholipid as main ingredients.

In the description, the oligo nucleic acid means a nucleic acid molecule having 10 to 50 nucleic acid bases and consisting of an RNA, a DNA, or a derivative of the RNA or the DNA. The oligo nucleic acid exhibits a bioactive effect in a cell and acts on a target DNA, a target RNA, or a protein which is an expression product of the target DNA or the target RNA to control or destroy the original cell function. A structure of the oligo nucleic acid is a single-stranded nucleic acid molecule, a nucleic acid molecule containing a double-stranded or a multi-stranded in one molecule, or a combination thereof (for example, a double-stranded nucleic acid formed of two single-stranded nucleic acid molecules).

### Background Art

As a method for inhibiting gene expression of a cell in a sequence specific manner, technologies using antisense nucleic acids have been known. The antisense nucleic acid is a single-stranded DNA derivative having a chain length of about 20 bases, and one of the most famous examples is phosphorothioate form, and this molecule can be administered alone in vivo. The antisense nucleic acid of phosphorothioate form can cleave a target RNA in a cell without forming a complex with a carrier such as a cationic liposome used in the present invention and exhibits a suppression of physiological function or a pharmaceutical effect in vivo. As the other compounds having RNA cleaving activity, a ribozyme and a DNA enzyme (deoxyribozyme or DNAzyme) have been known. However, these compounds are subject to degradation due to a nuclease since they have a natural nucleic acid structure, and have a characteristic of a remarkably low cellular uptake. Therefore, in order to effectively exhibit the bioactive effect in a cell, a carrier such as the cationic liposome is required.

Recently, an siRNA (small interfering RNA) has been found as an oligo RNA which exhibits the action of RNA interference (degradation of complementary strand mRNA). The siRNA is a double-stranded RNA having about 19 to 23 bases, generated by affecting an enzyme called Dicer which is present in a cell and has an RNAase III-like activity on a double-stranded RNA, and a structure thereof is protrusion type wherein each of strands has a single-stranded structure of 2 to 3 bases at its 3'end. Several derivatives of the siRNA have been reported, and a molecule having the 3' -overhang structure at least is reported to exhibit the satisfactory action of RNA interference. Also, a single-stranded RNA having a stem loop structure (double-strandedstructure in one molecule) is known to behave as a precursor of the siRNA (generally referred to as a short hairpin RNA or an shRNA). This molecule changes into a structure similar to that of the siRNA in processing by Dicer, thereby exhibits the action of RNA interference. As described above, in the case of nucleic acid molecules having a natural structure or derivatives thereof close to the natural structure, is very important how they are made to be introduced effectively and stably into a cell in view of practical application as a medicament.

It is known that a cationic liposome comprising a lipid which is positively charged in an aqueous solution is effective in introducing a nucleic acid such as a gene into a cell. It is likely that since the nucleic acid such as gene is charged negatively, the nucleic acid readily forms a complex with the cationic liposome and the complex is introduced into a cell by endocytosis of the cell. It is widely known that, in experiments using cultured cells, the antisense DNA and the siRNA can exhibit satisfactory effect only when introduced into the cell by means of the cationic liposome.

However, a complex comprising the above-described nucleic acid molecule and a cationic liposome, which can be practically used as a medicament capable of suppressing expression of target gene when it is administered to an animal, has not been found. One of primary reasons for the difficulty is considered to be a cytotoxicity of cationic liposomes.

In the meantime, it is known that a cationic liposome comprising a certain glycerol derivative and a phospholipid is effective in introducing a long chain double-stranded RNA such as poly(I):poly(C) having 50 to 10,000 base pairs into a cell (see, for example, WO94/19314, WO99/20283, WO99/48531). The complex of the cationic liposome has an average particle diameter of 100 nm or more and tends to be distributed predominantly in the reticuloendothelial systems of the liver and the spleen (see, for example, WO99/48531).

In the publications WO94/19314, WO99/20283, and WO99/48531, a double-stranded RNA having 50 or more base pairs is used. It is unknown whether or not the complex formed of the oligo nucleic acid having a chain length less than 50 base pairs and the cationic liposome disclosed in the above documents has a satisfactory stability and whether or not the complex thus formed can well be applied in vivo. Also, it is totally unknown whether or not the complex can exhibit safety and pharmacological efficacy suitable for practical use as a medicament when the complex is administered in vivo.

## Disclosure of the Invention

An object of the present invention is to primarily provide a complex of a cationic liposome with an oligo nucleic acid, which ensures satisfactory pharmacological efficacy as a medicament and safety.

The inventors of the present invention have conducted extensive researches to find that a complex obtainable from a certain cationic liposome and the oligo nucleic acid can be applied in vivo, exhibits pharmacological efficacy in vivo and thus be of practical use as a medicament, thereby accomplishing the present invention.

The present invention provides a complex of a cationic liposome with an oligo nucleic acid, wherein the liposome comprises 2-O-(2-diethylaminoethyl)carbamoyl-1,3-O-dioleoylglycerol and a phospholipid as main ingredients (hereinafter referred to as "the present liposome").

Also, the inventors have conducted further researches to find that an average particle diameter of particles of the complex can greatly influence a metabolic kinetics. That is, in the case of administrating intra-arterially or intravenously the complex having an average particle diameter exceeding 100 nm, the complex can be accumulated mainly in the liver, while the complex having an average particle diameter less than 100 nm, for example 80 nm, can escape from being captured in the reticuloendothelial systems such as the liver and the spleen, and can be widely distributed systemically. This means that the target sites for treatment of a disease with a medicament is expanded to other organs than the liver such as the lung and the circulatory system.

Therefore, the present invention provides a complex of the present liposome with an oligo nucleic acid, the complex having an average particle diameter of 100 nm or less.

Also, the present invention provides a pharmaceutical composition comprising a complex of the present liposome with an oligo nucleic acid, the complex being used for treatment or prevention of a disease caused by a target molecule (target DNA, target RNA, target protein) of the oligo nucleic acid.

## Brief Description of the Drawings

Fig. 1 is an aspect of dispersing a complex of Oligofectamine with a siRNA, the complex being prepared by a recommended method. An aggregate is observed in the complex of 10 μM in diameter.

Fig. 2 is an aspect of dispersing a complex with a siRNA, the complex being prepared using the present liposome.

Fig. 3 is a diagram of Western blotting showing the inhibition of Bcl-2 protein expression of A431 cells by adding the complex of bcl-2 siRNA and the present liposome.

Fig. 4 is a diagram of Western blotting showing the inhibition of Bcl-2 protein expression of A549 cells by adding the complex of bcl-2 siRNA and the present liposome.

Fig. 5 is a diagram showing a result of semi-quantitative RT-PCR that demonstrates the inhibition of bcl-2 mRNA expression of A431 cells by adding the complex of bcl-2 siRNA and the present liposome.

Fig. 6 is a diagram showing that the growth of A549 cells is inhibited by adding the complex of bcl-2 siRNA and the present liposome. The vertical axis of the graph indicates an absorbance which reflects the number of living cells.

Fig. 7 is a diagram showing a distribution of siRNA in foci of tumor 5 minutes after the administration of the complex of bcl-2 siRNA and the present liposome to the liver metastatic mice inoculated with A549 cells. The results of a hematoxylin and eosin staining (left), and an immunohistochemical staining using an anti-fluorescein antibody (right) are shown. The foci of A549 cell growth from mice to which the complex of the siRNA was administered (upper), and those to which naked siRNA was administered (lower) are shown. A positive signal appears in blue in the immunohistochemical staining.

Fig. 8 is a diagram showing the suppressive effect of the complex of bcl-2 siRNA and the present liposome on the growth of metastatic tumor when the complex was administered to the liver metastatic mice inoculated with A549 cells.

Fig. 9 is a diagram showing a survival curve of the liver metastatic mice inoculated with A549 cells, to which the complex of bcl-2 siRNA and the present liposome was administered.

Fig. 10 is a diagram showing a survival curve of the liver metastatic mice inoculated with A549 cells, to which the complex of bcl-2 siRNA and the present liposome was administered.

Fig. 11 is a diagram showing a dose dependency in life extension effect when the complex of bcl-2 siRNA and the present liposome was administered to the liver metastatic mice inoculated with A549 cells.

Fig. 12 is a diagram showing the effect of administration schedule of the complex of bcl-2 siRNA and the present liposome in the liver metastatic mice inoculated with A549 cells.

Fig. 13 is a diagram showing an effect of local administration of the complex of bcl-2 siRNA and the present liposome.

Fig. 14 is a diagram showing a comparison of tissue concentrations of siRNA in the plasma, liver, and spleen between the administration to mice of kinds of two complexes with siRNA, differing in particle diameter.

Fig. 15 is a diagram of Western blotting showing the inhibition of Bcl-2 protein expression of A431 cells by adding

the complex of bcl-2 antisense DNA and the present liposome.

Fig. 16 is a diagram of Western blotting showing the inhibition of Bcl-2 protein expression of A431 cells by adding the complex of bcl-2 DNA enzyme and the present liposome.

## Best Mode for Carrying out the Invention

Hereinafter, the present invention will be described in detail.

(1) The present liposome

One of components of the present liposome, 2-O-(2-diethylaminoethyl)carbamoyl-1,3-O-dioleoylglycerol (hereinafter referred to as "the glycerol derivative A"), is disclosed in WO94/19314 and has the following structural formula. Note that a synthesis method of the glycerol derivative A is shown in WO94/19314.

Structural Formula of the glycerol derivative A

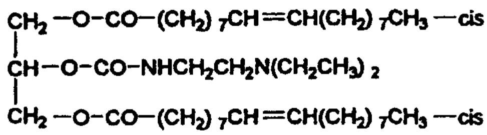

A phospholipid, which is another component of the present liposome, is not particularly limited insofar as the phospholipid is pharmaceutically acceptable. The phospholipids include phosphatidylcholine, phosphatidylethanolamine, phosphatidylinositol, phosphatidylserine, sphingomyelin, and lecithin. Preferred phospholipids include egg yoke phosphatidylcholine, egg yoke lecithin, soybean lecithin, and the like.

The formulating ratio of the glycerol derivative A to the phospholipid in the present liposome depends on the type of the phospholipid, and the ratio of the phospholipid to 1 part by weight of the glycerol derivative A may appropriately be in the range of 0.1 to 10 parts by weight, preferably in the range of 0.5 to 3 parts by weight, more preferably in the range of 1 to 2 parts by weight.

The present liposome is prepared by mixing the glycerol derivative A with the phospholipid and then dispersing the mixture in an aqueous solution. A machine such as a supersonic dispersing device, an emulsifying dispersion machine, and the like may appropriately be used for the dispersion processing.

(2) The oligo nucleic acid

The oligo nucleic acid means, as described in the foregoing, a nucleic acid molecule or its derivatives having 10 to 50 nucleic acid bases in one molecule and consisting of RNA and DNA. The oligo nucleic acid exhibits a bioactive effect in a cell and acts on a target DNA, a target RNA, or a protein which is an expression product of the target DNA or the target RNA to control or destroy the natural cell function. A structure of the oligo nucleic acid is a single-stranded nucleic acid molecule or a nucleic acid molecule containing double-stranded or multi-strand in parts one molecule or a combination thereof (for example, a double-stranded nucleic acid formed of two single-stranded nucleic acid molecules).

The oligo nucleic acids to be used in the present invention include, but not limited to, generally known DNAs, RNAs, and derivatives thereof. The specific oligo nucleic acids include nucleic acid molecules such as an siRNA, an shRNA, an antisense DNA, an antisense RNA, a DNA enzyme, a ribozyme, and an aptamer, which are well known and disclosed in literatures.

It is possible to design the oligo nucleic acid for each of almost all target RNA or DNA sequences, and the same is applicable to the case wherein the target is a protein. In both cases, it is possible to use the oligo nucleic acid in the present invention. The target molecules of the oligo nucleic acid include, but not limited to, an oncogene such as bcl-2, c-myc, and bcr-abl; a viral gene such as an HIV virus, a hepatitis C virus, and a hepatitis B virus; and an inflammation-related gene such as TNF-$\alpha$ and Fas. Also, a translation product of the genes in addition to a transcription product of the genes may be the target of the oligo nucleic acid. Further, not only the structural genes, but also a genome DNA which does not encode any proteins and an RNA molecule acting for regulating protein expression, such as an RNA molecule acting for maturation of a tRNA precursor having a splicing sequence and an RNA molecule including a micro RNA, are included in the target.

The oligo nucleic acid to be used in the present invention is not limited to the naturally occurring type, and it is possible to modify at least a part of a nucleotide, such as a sugar and a phosphate backbone constituting the nucleotide, in order to enhance in vivo stability such as a nuclease resistance. In the case of modification, preferable modification may be a modification of 2'position of the sugar, a modification of positions other than 2'position of the sugar, a modification of the phosphate backbone of the oligo nucleic acid, or the like. The modifications of 2' position of the sugar include a replacement with OR, R, R'OR, SH, SR, $NH_2$, NHR, $NR_2$, $N_3$, CN, F, Cl, Br, I, and the like. "R" represents alkyl or aryl, preferably an alkyl group having 1 to 6 carbon atoms, and "R'" represents alkylene, preferably alkylene having 1 to 6 carbon atoms. The modified products of the other parts of the sugar include a 4'thio form, and the like. The modified products of the phosphate backbone of the oligo nucleic acid include a phosphorothioate form, a phosphorodithioate form, an alkylphosphonate form, a phosphoroamidate form, and the like.

Though the oligo nucleic acid of the present invention has 10 to 50 nucleic acid bases in one molecule, it is also possible to use the oligo nucleic acid having 15 to 35 nucleic acid bases or 18 to 25 nucleic acid bases.

It is possible to synthesize the oligo nucleic acid by using a phosphoamidite method or a triester method, which are known to person skilled in the art, in a solid phase or a liquid phase. The most conventional mode is a solid phase synthesis by the phosphoamidite method, and it is possible to perform the synthesis by using a nucleic acid automatic synthesizer or manually. After completion of the synthesis in solid phase, a removal from the solid phase, a removal of protective group, and a purification of the product are performed. It is desirable to obtain a nucleic acid having a purity of 90 % or more, preferably 95 % or more by purification.

(3) Complex

A ratio of the oligo nucleic acid to the present liposome in the formation of the complex varies depending on the type of the oligo nucleic acid, and a proportion of the present liposome to 1 part by weight of the oligo nucleic acid may suitably be 0.01 to 100 parts by weight, preferably 1 to 30 parts by weight, and more preferably 10 to 20 parts by weight.

Since the present liposome is charged positively at a pH lower than neutral, the present liposome readily forms a complex with the oligo nucleic acid which is negatively charged. Therefore, the complex of the present invention can be produced by: dispersing the glycerol derivative A and a phospholipid in an aqueous solution to form the present liposome, adding the oligo nucleic acid to the present liposome, and dispersing the mixture; or by dispersing the glycerol derivative A, a phospholipid and the oligo nucleic acid in an aqueous solution.

The aqueous solutions used for forming the complex include water for injection, distilled water for injection, an electrolytic solution such as saline; and a carbohydrate solution such as a glucose solution and a maltose solution.

The dispersion process may be performed by using a homomixer, a homogenizer, an ultrasonic dispersion machine, an ultrasonic homogenizer, a high pressure emulsifying dispersion machine, Microfluidizer (brand name), Nanomizer (brand name), Altimizer (brand name), DeBEE2000 (brand name), a manton-gaulin type high pressure homogenizer, or the like. The conditions, time and temperature of the process may be selectable. The dispersion process can be performed by doing several steps, which include coarsely dispersing step.

The complex according to the present invention is not particularly limited in average particle diameter, but an appropriate particle size may be not more than 300 nm. The average particle diameter of the complex may preferably be 10 to 100 nm, more preferably 20 to 80 nm, yet more preferably 30 to 50 nm. It is possible to adjust the size from 10 to 300 nm by changing the dispersion conditions.

(4) Usage of the complex

The complex of the present invention can be introduced into a cell and exhibits a bioactive effect and a pharmacological effect on a target RNA, DNA, or protein. By using the complex of the present invention, it is possible to exhibit a targeting effect on an RNA, DNA, or protein in cells in vivo. Due to such an intracellular targeting, it is possible to cause degradation of target RNA, regulation of expression of target DNA, and dysfunction by binding to a target protein, and, ultimately, it is possible to cause suppression of expression or dysfunction of a protein which is a target for disease treatment. These effects enable functions of the oligo nucleic acid to be used for pharmaceutical purposes. The complex of the present invention is useful as an ingredient of a pharmaceutical composition which is capable of treating or preventing various diseases as a medicament of the oligo nucleic acid.

(5) Pharmaceutical composition

The present invention provides a pharmaceutical composition characterized by containing a complex of the oligo nucleic acid and the present liposome, i.e., the present invention provides a pharmaceutical composition to be safely administered to humans.

The pharmaceutical composition of the present invention may be in the form of a liquid formulation (injections,

drops; or the like) obtainable by dispersing the complex into an aqueous solution or a freeze-dried preparation of the liquid formulation. In the case of the liquid formulation, a concentration at which the complex is present in the liquid formulation may suitably be from 0.001 to 25 % (w/v), preferably from 0.01 to 5 (w/v), more preferably from 0.1 to 2 % (w/v).

The pharmaceutical composition of the present invention may contain a pharmaceutically acceptable additives such as an emulsifying auxiliary agent, a stabilizer, a tonicity agent, and a pH adjuster in an appropriate amount. More specifically, the additives include aliphatic acids having 6 to 22 carbon atoms (for example, caprylic acid, capric acid, lauric acid, myristic acid, palmitic acid, stearic acid, oleic acid, linolic acid, arachidonic acid, and docosahexaenoic acid) and pharmaceutically acceptable salts thereof (for example, a sodium salt, a potassium salt, and a calcium salt); the emulsifying auxiliary agents such as albumin and dextran; the stabilizer such as cholesterol and phosphatidic acid; the tonicity agents such as sodium chloride, glucose, maltose, lactose, sucrose, and trehalose; the pH adjusters such as hydrochloric acid, sulfuric acid, phosphoric acid, acetic acid, sodium hydrochloride, potassium hydrochloride, and triethanolamine; and the like.

It is possible to add the pharmaceutically acceptable additive before or after the dispersion at an appropriate time of the process.

It is possible to provide the pharmaceutical composition as a medicament by performing satisfactory bacteria elimination or sterilization.

A freeze-dried preparation of the pharmaceutical composition of the present invention is prepared by freeze-drying the complex obtained by the above dispersion processing. The freeze-drying is achieved by a conventional method. For example, after sterilizing the complex obtained by the above dispersion processing, the complex is dispensed into vial containers each in a predetermined amount, and then the complex is subjected to preliminary freezing at about -40 to -20 °C for about 2 hours, followed by a first drying at about 0 to 10°C under a reduced pressure and a secondary drying at about 15 to 25 °C under a reduced pressure. Then, the air inside the vial containers is ordinarily replaced with a nitrogen gas, and the vial containers are finally capped to give a freeze-dried preparation of the complex.

The freeze-dried preparation of the pharmaceutical composition of the present invention is ordinarily reconstituted by adding thereto an appropriate solution (reconstituting solution) to be used. The reconstituting solutions include water for injection, saline, and the other standard infusions. An amount of the reconstituting solution may vary depending on an usage and the like, and is not particularly limited, but an appropriate amount may be 0.5 to 2 times the amount of the original liquid formulation before the freeze-drying or not more than 500 mL.

The pharmaceutical composition of the present invention may preferably be administrated with an administration unit form, and can be administered by intravenous administration, intra-arterial administration, oral administration, intra-tissue administration, transdermal administration, transmucosal administration, or rectal administration to animals including humans. Particularly, the intravenous administration, the transdermal administration, and the transmucosal administration are preferred. It is also possible to perform local administration as is the case of intra-tissue administration for cancer treatment. The pharmaceutical composition is administered in formulations appropriate for the above-mentioned types of administration, such as various injections, an oral preparation, drops, an inhalant, eye drops, an ointment, a lotion, and a suppository.

For example, it is desirable to change the dose of the pharmaceutical compositions of the present invention as a medicament in consideration of a drug, a formulation, a patient's conditions such as age and body weight, a route for administration, and characteristics and a seriousness of a disease, and usually the dose is in the range of 0.1 mg/day/person to 10 g/day/person as an oligo nucleic acid for an adult, preferably in the range of 1 mg/day/person to a several grams/day/person. This dose may vary depending also on a type of target disease, an administration style, and a target molecule. Therefore, the dose may be lower than that or must be increased in some cases. The composition may be administered once or several times a day and at intervals of 1 to several days.

## Examples

Hereinafter, the oligo nucleic acid whose target molecule is an mRNA of bcl-2 will be described by way of examples, but the present invention is not limited by the examples at all. A sequence of the oligo nucleic acid is described below.

SEQ ID No. 1 (sense strand of bcl-2 siRNA)

5'-GUGAAGUCAACAUGCCUGCdTdT-3'

SEQ ID No. 2 (antisense strand of bcl-2 siRNA)

5'-GCAGGCAUGUUGACUUCACdTdT-3'

SEQ ID No. 3 (sense strand of luciferase siRNA)

5'-CUUACGCUGAGUACUUCGAdTdT-3'

SEQ ID No. 4 (antisense strand of luciferase siRNA)

5'-UCGAAGUACUCAGCGUAAGdTdT-3'

SEQ ID No. 5 (bcl-2 antisense DNA)

5'-TsCsTsCsCsCsAsGsCsGsTsGsCsGsCsCsAsT-3'

SEQ ID No. 6 (reverse sequence of bcl-2 antisense DNA / negative control)

5'-TsAsCsCsGsCsGsTsGsCsGsAsCsCsCsTsCsT-3'

SEQ ID No. 7 (bcl-2 DNA enzyme)

5'-GsGsCsCsAsCsAsAGsGCsTAsGCsTAsCAsACsGACsCsTsCsCsCsCsC-3'

SEQ ID No. 8 (mutant of bcl-2 DNA enzyme / negative control)

5'-GsGsCsCsAsCsAsAGsGCsGAsCCsTAsCAsACsGACsCsTsCsCsCsCsC-3'

SEQ ID No. 9 (sense strand of bcl-2 siRNA)

5'-GUGAUGAAGUACAUCCAUUdTdT-3'

SEQ ID No. 10 (antisense strand of bcl-2 siRNA)

5'-AAUGGAUGUACUUCAUCACdTdT-3'

SEQ ID No. 11 (sense strand of bcl-2 siRNA)

5'-GAGAUAGUGAUGAAGUACAUCCAUUdTdT-3'

SEQ ID No. 12 (antisense strand of bcl-2 siRNA)

5'-AAUGGAUGUACUUCAUCACUAUCUCdTdT-3'

A part of the above-mentioned oligo nucleic acids was synthesized by Dharmacon, Inc. (Colorado, USA), Japan Bio Service Co., Ltd. (Saitama prefecture), and Hokkaido System Science Co., Ltd. (Hokkaido). Note that 2 bases at 3'end of the siRNA are of a 2'deoxy form. The above-mentioned antisense DNAs and DNA enzymes are 2' deoxy forms, but a phosphate backbone of the sites represented by "s" is modified into a phosphorothioate form.

Preparation Example 1: Synthesis of hcl-2 siRNA

Synthesis of each of siRNAs of SEQ ID No. 1, 2, 9, and 10 was performed by using an automatic DNA synthesizing device (Expedite 8909; product of Applied Biosystems) and by a conventional amidite method (Nucleic Acid Research 1984, 12, 4539.). Cleavage from CPG was conducted by using a concentrated ammonium hydroxide-ethanol (3/1) mixture solution, followed by deprotection by leaving in the same mixture solution at 55 °C (18 hours). Then, deprotection of a silyl group at 2'position was performed by using a 1 M tetrabutylammonium fluoride solution in tetrahydrofurane at a room temperature (20 hours). The obtained oligoribonucleotide was purified by a reverse phase chromatography. Then, deprotection of a DMTr group at 5' position was performed in an 80% acetic acid solution at a room temperature (30 minutes), followed by purification by ion exchange chromatography. Oligoribonucleotide obtained after desalting was subjected to a capillary gel electrophoresis and it was determined that 90% or more thereof is a full-chain length substance.

Reference Example 1: Preparation of the present liposome

Twenty g of maltose dissolved in 100 mL of water for injection was added to 1.2 g of the glycerol derivative A and 2 g of high purity yoke lecithin, and then the mixture was dispersed for 30 minutes by using a small emulsifying dispersion machine for experiments. The amount of the dispersion was adjusted to 200 mL using water for injection to obtain a solution containing the present liposome at 16 mg/mL.

Example 1 : Preparation of complex of siRNA and the present liposome (1)

One mL of an siRNA solution having a concentration of 20 μM (268 μg/mL) was prepared by adding 900 μL of a 10% maltose solution to 100 μL of a 200 μM double-stranded siRNA solution (SEQ ID Nos. 1 and 2). Also, 1 mL of a solution containing the present liposome at 4.3 mg/mL was prepared by adding 732 μL of a 10% maltose solution to 268 μL of the solution containing the present liposome at 16 mg/mL described in Reference Example 1. One mL of the siRNA solution was gradually added to 1 mL of the solution containing the present liposome at 4.3 mg/mL with stirring. By the above procedure, an siRNA complex having an siRNA of the final concentration of 10 μM was obtained. An average particle diameter of the complex was measured by using a particle diameter measurement device (NICOMP 380ZLS; product of Particle Sizing Systems, USA) which employs a dynamic light scattering method and by using Vesicle mode as a measurement mode (average particle diameter measurements described hereinafter were performed by using the same device and the same measurement mode), and the detected average particle diameter was 181 nm. The complex was further dispersed by using a water bath ultrasonic device to obtain a complex having an average particle diameter of 155 nm.

The complex of the present liposome with a double-stranded RNA consisting of the SEQ ID Nos. 9 and 10and the complex of the present liposome with a double-stranded RNA consisting of the SEQ ID Nos. 11 and 12 were prepared in the same manner as described above.

Example 2: Preparation of complex of siRNA and the present liposome (2)

Distilled water in an amount of 8 mL was added to 300 mg of the glycerol derivative A, 500 mg of a high purity yoke lecithin, and 1 g of maltose, followed by vigorously stirring for dispersion. The dispersion was then irradiated with ultrasonic wave in an ultrasonic crusher for 15 minutes under cooling by ice water. The amount of the dispersion after the irradiation was adjusted to 10 mL using a distilled water, followed by filtering using a 0.22 μm filter to obtain a solution of the present liposome (80 mg/mL) having an average particle diameter of 44.4 nm. To 1 mL of thus-obtained liposome solution, 2.61 mL of a 15.3 % maltose solution was added, followed by stirring. This mixture was again cooled on ice, and 1.39 mL solution containing the 3.6 mg/mL siRNA (SEQ ID Nos. 1 and 2) was poured into it under ultrasonic irradiation. Then, supersonic irradiation was performed to obtain a 1 mg/mL complex with an siRNA having an average

particle diameter of 38.5 nm.

Example 3: Preparation of complex of antisense DNA and the present liposome

By adding 900 μL of 10 % maltose solution to 100 μL of antisense DNA (SEQ ID No. 5) solution having the concentration of 200 μM, 1 mL of antisense DNA solution having a concentration of 20 μM (114 μg/mL) was prepared. Also, by adding 886 μL of 10 % maltose solution to 114 μL of the solution containing the present liposome at 16 mg/mL described in Reference Example 1, 1 mL of a solution containing the present liposome at 1.8 mg/mL was prepared. One mL of the antisense DNA solution was gradually added to 1 mL of the solution containing the present liposome at 1.8 mg/mL with stirring. Thus, a complex with antisense DNA having antisence DNA of an final concentration of 10 μM was obtained by the above operation. The complex particles were dispersed using the water bath ultrasonic device to obtain complex particles having an average particle diameter of 120.5 nm.

Example 4 : Preparation of complex of DNA enzyme and the present liposome

By adding 900 μL of 10 % maltose solution to 100 μL of DNA enzyme (SEQ ID No. 7) solution having the concentration of 200 μM, 1 mL of DNA enzyme solution having a concentration of 20 μM (191 μg/mL) was prepared. Also, by adding 809 μL of 10% maltose solution to 191 μL of the solution containing the present liposome at 16 mg/mL described in Reference Example 1, 1 mL of the solution containing the present liposome at 3.1 mg/mL was prepared. One mL of the DNA enzyme solution was gradually added to 1 mL of the solution containing the present liposome at 3.1 mg/mL with stirring. Thus, a complex with DNA enzyme having a DNA enzyme of an final concentration of 10 μM was obtained by the above operation. The complex particles were dispersed using the water bath supersonic device to obtain complex particles having an average particle diameter of 135.1 nm.

Comparative Example 1 : Comparison of hemolyzing properties of commercially available carriers and the present liposome

Since many of cationic carriers in general have a surfactant activity, a comparison of a hemolyzing property was carried out among various commercially available carriers and the present liposome. Used as the commercially available carriers were Oligofectamine (brand name), Trans IT-TKO (brand name) Trans Messenger (brand name) Lipofectamine 2000 (brand name), Lipofectamine (brand name), and DMRIE-C (brand name).

After blood drawn from rat and treated with heparin was centrifuged at 3, 000 rpm for 10 minutes, a blood plasma of the top layer and a white blood layer were eliminated. To thus-obtained red blood cells, an injectable saline in an amount twice that of the red blood cells was added to be mixed with the red blood cells, followed by centrifuging at 3,000 rpm for 5 minutes. This operation was repeated twice, and thus-obtained red blood cells were diluted to $1 \times 10^8$ cells/mL using the saline for injection to obtain a suspension with red blood cells. One hundred μl of the solution containing the present liposome diluted to an appropriate concentration using saline or a commercially available carrier solution was added to 850 μL of a buffer for measurement (74 mMNaCl, 147 mM Sucrose, 6 mM Glucose, 20 mM Tris-HCl, pH 7.2), followed by a preliminary heating at 37 °C. Then 50 μL of the suspension with red blood cells was added to the solution containing the present liposome or commercially available carrier solution, followed by incubation at 37 °C for 45 minutes. The reaction solution was centrifuged at 3, 000 rpm for 3 minutes, and then an absorbance of a supernatant at 543 nm was measured and a hemolysis (%) was calculated using Expression 1.

Expression 1

$$\text{Hemolysis (\%)} = \frac{OD_{exp} - OD_{blank}}{OD_{100} - OD_{blank}} \times 100$$

$OD_{exp}$ : Absorbance of a sample which treated a test substance.
$OD_{100}$ : Absorbance of a sample which hemolyzed fully by treated 0.2% TritonX100.
$OD_{blank}$ : Absorbance of a sample which treated a saline.

Carrier concentrations at which 40 % of the red blood cells from rats were hemolyzed are shown in Table 1.

Thus, the present liposome has a remarkably low hemolyzing property as compared with the commercially available carriers.

[Table 1]

| carrier | concentration of a carrier for causing hemolysis to 40% (μ g/mL) |
|---|---|
| the liposome of the present invention | >1000 |
| the commercial article A | 16.6 |
| the commercial article B | 24.9 |
| the commercial article C | 72.3 |
| the commercial article D | 53.7 |
| the commercial article E | 15.9 |
| the commercial article F | 89.9 |

Comparative example 2: Comparison of cytotoxicities of commercially available carriers and the present liposome

Human umbilical vein endothelial cells (product of Sanko Junyaku Co., Ltd.) were seeded on a 96-well plate in such a way that about 3,000 cells were placed on each of the wells and then cultured overnight. A complex of the present liposome with the siRNA (SEQ ID Nos. 3 and 4) targeting a firefly luciferase gene or complexes of the commercially available carriers with the same siRNA were added to the respective wells in one tenth the volume of the cell suspension. After culturing for 72 hours, Cell Counting Kit 8 (WST-8, product of Dojindo Laboratories) was added, and the number of living cells was determined by measuring absorbance. Note that a mixing ratio of the siRNA and the carrier (weight ratio) was 1:16, and the complex preparation was performed in accordance with an attached protocol of each of the reagents.

Concentrations of the carriers that inhibited proliferation of the human umbilical vein endothelial cells by 50 % are shown in Table 2. It was revealed that the complex prepared by using the present liposome was low in toxicity as compared to the complexes formed from the commercially available carriers (the carrier used in Comparative Example 1).

[Table 2]

| carrier | concentration of a carrier for inhibiting cell growth to 50% (μg/mL) |
|---|---|
| the liposome of the present invention | >100 |
| the commercial article A | 12.4 |
| the commercial article B | 3.2 |
| the commercial article C | 76.8 |
| the commercial article D | 48.2 |
| the commercial article E | 7.6 |
| the commercial article F | 19.1 |

Comparative Example 3: Comparison of morphologies of complexes of Oligofectamine and siRNA and complexes of the present liposome and siRNA

A complex of Oligofectamine (product of Invitrogen Corporation) with the bcl-2 siRNA (SEQ ID Nos. 1 and 2) was formed. The siRNA and the Oligofectamine were mixed at a mixing ratio which was 3 μL of the Oligofectamine to 60 pmole of the siRNA (double-stranded) in accordance with the manual by Tuschl et al (http://www.mpibpc.gwdg.de/abteilungen/100/105/siRNA.html). An annealing was performed on the siRNA in accordance with the manual. According to this method, complexes with siRNA of 10 μM, 3 μM, and 1 μM were prepared and morphologies thereof were compared. Note that the concentration of each of the complexes with siRNA were indicated by a molar concentration of the double-stranded siRNA contained in the complex. A slight white turbidity was observed in the 1 μM complex; an aggregate was observed in the 3 μM complex; and rough and large particles were visually recognized in the 10 μM complex (Fig. 1).

On the other hand, complexes having an final concentration of 10 μm, 3 μM, and 1 μM were prepared in the same manner as in Example 1 by using the present liposome and forms thereof were compared (Fig. 2).

Though the visible aggregate was formed in complex in the case of Oligofectamine at 3 μM or more, no apparent

aggregate was observed in the 10 μM complex prepared by using the present liposome.

Test Example 1 : Effect of siRNA using the present liposome (1) - protein level -

In each of petri dishes having a diameter of 6 cm, 2 x $10^5$A431 cells (human epidermoid carcinoma cells) were seeded and cultured overnight. On the following day, the culture media were replaced with fresh culture media (2.7 mL), and then 0. 3 mL of a complex solution prepared by the method of Example 1 and containing the bcl-2 siRNA (SEQ ID Nos. 1 and 2) was added to the culture media. The concentration of the complex solution was adjusted to 10 times that of the final concentration using a 10 % maltose solution to be added to the culture media. Meanwhile, a complex containing the siRNA (SEQ ID Nos. 3 and 4) for the firefly luciferase was prepared as a negative control, and was added to the culture media. The cells were collected 72 hours after the addition of the siRNAs, and the collected cells were dissolved by using a cell dissolving buffer solution [50 mM Tris-HCl (pH8.0); 150 mM NaCl; 1% NP-40; 1 mM PMSF; 1x Complete™ (product of Roche Diagnostics K.K.)]. The samples whose total protein contents was adjusted to 15 μg/lane were subjected to an SDS-polyacrylamide gel electrophoresis (5-20 % gradient gel). The isolated protein was transferred onto a PVDF film using a semidry blotting device, and then a Western blotting analysis was performed by using an anti-human Bcl-2 antibody (M0887; product of DAKO AG) or an anti-actin polyclonal antibody (sc-1616; product of Santa Cruz Biotechnology, Inc.). A peroxidase-labeled anti-mouse IgG antibody or a peroxidase-labeled anti-goat IgG antibody was used as a secondary antibody to detect the target protein with the use of a chemiluminescence reagent (Renaissance Luminol; product of Daiichi Pure Chemicals Co., Ltd.).

As shown in Fig. 3, the bcl-2 siRNA suppressed the expression of Bcl-2 protein in a concentration dependent manner, while such suppression was not observed with the siRNA for luciferase which was the negative control. Also, no influence was exerted on expression of beta-actin which was another endogenous protein. From this result, it was revealed that the bcl-2 siRNA suppresses the expression of the Bcl-2 protein in the sequence specific manner, and the present liposome is effective in causing the siRNA to exhibit its effect in vitro. The bcl-2 siRNA formed from the SEQ ID Nos. 11 and 12 also suppressed expression of the Bc1-2 protein in a concentration dependent manner.

Test Example 2: Effect of siRNA using the present liposome (2) - protein level -

The suppressive effect on Bcl-2 protein expression in A549 cells (human lung epithelial carcinoma cells) was examined in the same manner as in Test Example 1. As shown in Fig. 4, effectiveness of the present liposome was exhibited.

Test- Example 3: Effect of siRNA using the present liposome (3) - mRNA level -

In order to examine whether the suppression of protein expression by the siRNA with the present liposome is caused by the suppression of mRNA expression, the level of mRNA expression of the target gene was determined. A431 cells were treated in the same manner as in Test Example 1 and collected 24 hours after the addition of the complex, and then total RNAs were extracted by using ISOGEN (product of Nippon Gene Co., Ltd.). Then, using an aliquot of the total RNA as a template, a cDNA was synthesized by a reverse transcription reaction using THERMOSCRIPT RT-PCR system (product of Invitrogen Corporation). Further, a certain amount of the reverse transcription reaction solution was used as a template for a PCR reaction to perform a semi-quantitative determination of the bcl-2 mRNA level by a detection and quantification system (Light Cycler, product of Roche Diagnostics K.K.) using the capillary PCR method.

Consequently, treatment with 10 nM of the bcl-2 siRNA complex for 24 hours reduced the level of bcl-2 mRNA to about 20% of that attained by an addition of the negative control (Fig. 5). Thus, it was confirmed that the suppressive effect of the complex of the liposome with siRNA on the protein expression is caused by the suppression of mRNA expression.

Test Example 4: Effect of si RNA using the present liposome (4) - cytostatic effect -

A549 cells were cultured on a 96-well plate at a density of 1 $\times$ $10^3$ cells/well, and 1/10 volume of complex solution prepared by the method described in Test Example 1 and containing the bcl-2 siRNA (SEQ ID Nos. 1 and 2) was added to the culture media on the following day. Meanwhile, a complex containing the siRNA (SEQ ID Nos. 3 and 4) for the firefly luciferase was prepared as a negative control, and was added to the culture media. On 6th day after the addition of each of the complexes, the number of cells was measured by using Cell Proliferation Kit 1 (product of Roche Diagnostics K.K.) . As shown in Fig. 6, the A549 cells in which the Bcl-2 protein expression was suppressed by the complex with bcl-2 siRNA showed suppressed proliferation in a concentration dependent manner.

Test Example 5: Delivery of siRNA to carcinoma foci using the present liposome

A549 cells ($10^6$ cells/mouse) were inoculated in the spleen of nude mice (BALB/c, nu/nu, male, 5 weeks old), and then the spleen was removed 10 minutes after the inoculation. By this operation, the A549 cells were allowed to migrate to the liver to form metastatic foci. After 5 weeks from the operation, a complex of the siRNA (SEQ ID Nos. 1 and 2) with fluorescein labeling at 5'end and the present liposome was prepared in the same manner as in Example 1 to be administered to the mice intravenously from the tail vein in an amount of 5 mg/kg. An average particle diameter of the administered complex was 235.4 nm. The liver was removed from the mouse after 5 minutes and 1 hour from the administration of the complex to be fixed in a PBS containing 4 % paraformaldehyde solution. Then, the liver was subjected to paraffin embedding and sectioned at 3 microns. Immunohistochemical staining using an alkaline phosphatase labeled anti-fluorescein antibody (D5101, product of DAKOAG) was performed on the tissue sections to examine distribution of the siRNA in tissue. For the color development to demonstrate the alkaline phosphatase activity, BCIP/NBT colorimetric substrate (product of DAKOAG) was used. As a result, a positive signal was recognized diffusely at the sites of theA549 cell proliferation. However, in the case of administrating naked fluorescein labeled siRNA, any signal was not observed in carcinoma foci. Shown in Fig. 7 are consecutive sections of the carcinoma foci in the mouse liver after 5 minutes from the complex of the administration. Shown in the upper part of Fig. 7 is the carcinoma foci of the mouse to which the complex was administered, and shown in the lower part of Fig. 7 is the carcinoma foci of the mouse to which the naked siRNA was administered. A wide range of the carcinoma foci on the upper part, from which the fluorescein was detected, was stained in blue. Therefore, potentiality of the delivery of the siRNA to tumor cells in foci by the use of the present liposome was suggested.

Test Example 6: Effect of siRNA using the present- liposome (5) - In vivo effect -

A549 cells ($10^6$ cells/mouse) were inoculated in the spleen of nude mice (BALB/c, nu/nu, male, 5 weeks old), and then the spleen was removed 10 minutes after the inoculation. From 6th day to 45th day after the inoculation, 10 mg/kg of a complex prepared by the method described in Example 1 and containing the bcl-2 siRNA (SEQ ID Nos. 1 and 2) was administered intravenously once a week (total 6 times) or three times a week (total 18 times). Ten % maltose solution was administered to a control group three times a week (total 18 times). Gross appearance and liver weight of in both groups are shown in Fig. 8 and Table 3.

[Table 3]

| group | liver weight (g) | | | | |
|---|---|---|---|---|---|
| | No.1 | No.2 | No.3 | No.4 | Mean |
| control | 2.67 | 4.50 | 2.29 | - | 3.15 |
| siRNA/carrier complex, 10mg/kg, once/week | 1.91 | 2.35 | 2.26 | - | 2.17 |
| siRNA/carrier complex, 10mg/kg, 3 times/week | 2.26 | 1.67 | 1.76 | 1.43 | 1.78 |

Test Example 7-1: Effect of siRNA using the present liposome (6) - In vivo effect -

A549 cells ($10^6$ cells/mouse) were inoculated in the spleen of nude mice (BALB/c, nu/nu, male, 5 weeks old), and then the spleen was removed 10 minutes after the inoculation. From 6th day to 45th day after the inoculation, a complex prepared by the method described in Example 1 and containing the bcl-2 siRNA (SEQ ID Nos. 1 and 2) was administered intravenously once a week (total 6 times) or three times a week (total 18 times). Ten % maltose solution was administered to a control group three times a week (total 18 times). Fig. 9 is a graph showing the number of survived mice until 100 days after the inoculation.

By subjecting Kaplan Meier curves of the drug administration group and the control group to the generalized Wilcoxon test, a statistically significant difference was confirmed ($P < 0.01$).

Test Example 7-2: Effect of siRNA using the present liposome (7) - In vivo effect -

A549 cells ($10^6$ cells/mouse) were inoculated in the spleen of nude mice (BALB/c, nu/nu, male, 5 weeks old), and then the spleen was removed 10 minutes after the inoculation. From 6th day to 44th day after the inoculation, a complex prepared by the method described in Example 1 and containing the bcl-2 siRNA (SEQ ID Nos. 9 and 10) was administered intravenously twice a week (total 12 times). The administration dose was 10 mg/kg weight as the siRNA. Ten % maltose solution was administered to a control group twice a week (total 12 times) . Fig. 10 is a graph showing

the number of survived mice until 100 days after the inoculation.

By subjecting Kaplan Meier curves of the bc1-2 siRNA administration group and the control group to the generalized Wilcoxon test, a statistically significant difference was confirmed ($P < 0.01$).

Test Example 7-3: Effect of siRNA using the present liposome (8) - dose dependency -

A549 cells ($10^6$ cells/mouse) were inoculated in the spleen of nude mice (BALB/c, nu/nu, male, 5 weeks old), and then the spleen was removed 10 minutes after the inoculation. From 6th day to 44th day after the inoculation, a complex prepared by the method described in Example 1 and containing the bcl-2 siRNA (SEQ ID Nos. 9 and 10) was administered intravenously twice a week (total 12 times). The dosage was 1 mg/kg weight, 3 mg/kg weight, or 10 mg/kg weight as the siRNA. Ten % maltose solution was administered to a control group twice a week (total 12 times). The results are shown in Fig. 11.

By subjecting Kaplan Meier curves till the 69 day after the tumor inoculation of the 1 mg/kg weight administration group, the 3 mg/kg weight administration group, the 10 mg/kg weight administration group, and the control group to the generalized Wilcoxon test, a statistically significant difference was confirmed ($P < 0.05$, $P < 0.01$, $P < 0.01$).

Test Example 7-4: Effect of siRNA using the present liposome (9) - study on consecutive administration schedule -

A549 cells ($10^6$ cells/mouse) were inoculated in the spleen of nude mice (BALB/c, nu/nu, male, 5 weeks old), and then the spleen was removed 10 minutes after the inoculation. From 5th day to 10th day after the inoculation, a complex prepared by the method described in Example 1 and containing the bcl-2 siRNA (SEQ ID Nos. 9 and 10) was administered intravenously five times a week (total 5 times or 12 times) . The dosage was 10 mg/kg weight as the siRNA. Ten % maltose solution was administered to a control group five times a week (total 12 times). The results are shown in Fig. 12.

On the day 69 after the tumor inoculation, all the 10 mice survived in each of the five times administration group and the 12 times administration group. By subjecting Kaplan Meier curves till the day 69 after the tumor inoculation of the drug administration groups and the control group to the generalized Wilcoxon test, a statistically significant difference was confirmed ($P < 0.01$).

Test Example 7- Effect of local administration using the present liposome - In vivo effect **-**

To the right side of each of nude mice (BALB/c, nu/nu, male, 5 weeks old), $2.5 \times 10^6$ of PC-3 cells suspended in 100 $\mu$L of a PBS were inoculated subcutaneously. From 7th day to 18th day after the inoculation, each of complexes prepared by the method described in Example 1 and containing the bcl-2 siRNAs (SEQ ID Nos. 1 and 2, SEQ ID Nos. 9 and 10) was administered subcutaneously near the tumor site 5 times a week (total 10 times). The dosage was 0.1 mg/mouse as the siRNA. Ten % maltose solution was administered to a control group 5 times a week (total 10 times). A volume of the tumor was determined by formula "volume = minor axis x minor axis x major axis $\div$ 2" assuming the tumor as an ellipsoid. The result is shown in Fig. 13.

The increase in the tumor volume was suppressed from 14th day to 36th day after the tumor inoculation in each of the complex administration groups as compared to the control group with a statistically significant difference ($P < 0.01$, Dunnett's test).

Test Example 8: Intra-tissue concentration of siRNA/the present liposome complex

(1) Synthesis of tritium labeled siRNA

In order to synthesize an siRNA internally labeled with tritium ($^3H$), a $^3H$ labeledATP (NET-420, product of Perkin Elmer Life Science) was taken up as a part of a substrate during T7 polymerase transcription reaction by using Silencer™ siRNA construction kit (product of Ambion, Inc.). The $^3H$ labeled ATP was added in an amount of 3.7 Mbq (2.7 nmole) per 20 $\mu$L of a reaction solution, and a double-stranded siRNA was synthesized in accordance with an attached document of the kit. After that, the reaction solution was treated with phenol/chloroform to eliminate protein, and then unreacted monomers were eliminated by using G-25 spin column (product of Pharmacia Corp.). The $^3H$ labeled double-stranded siRNA thus-obtained was subjected to electrophoresis by using a 12% acrylamide gel and then to a calibration using an image analyzer (BAS2500; product of Fuji Photo Film Co., Ltd.), and it was confirmed that the siRNA has the size of 20 to 21 base pairs and does not include any monomer.

(2) Preparation of complex

A complex with siRNA having an average particle diameter of 187.5 nm was prepared by the method described in Example 1, and an complex with siRNA having an average particle diameter of 43.3 nm was prepared by the method described in Example 2. Each of the complexes was prepared by using a mixture of the $^3H$ labeled substance and unlabeled substance, and a specific activity was adjusted to 1,000 dpm/μg to 2,000 dpm/μg.

(3) Measurement of intra-tissue concentration

Each of the complexes different in size was administered to mice (ddy, 4 weeks old, male, provided by SLC, Inc.) via the tail vein in an amount of 5 mg/kg (siRNA concentration) . Blood was sampled from each of the mice under anesthesia by ether at 5 minutes, 15 minutes, and 60 minutes after the administration, and then the mice were left to die. Heparin was used as an anticoagulant agent for obtaining plasma. Then, the liver, the lung, the heart, the spleen, and the kidney were removed to measure a wet weight of each of the organs. To each of vials containing the removed organs, 1 mL of a tissue dissolving agent (Solbavle™; product of Packard Instrument Company) was added, and then the tissues were dissolved by shaking at 40°C overnight (a portion of each of the liver and the kidney obtained by weighing was used for the dissolution) . To each of the samples showing a staining caused by the red blood cells, 100 μL of a 30 % hydrogen peroxide solution was added for discoloration, and then 10 mL of a scintillation cocktail (Hionic Fluor™, product of Packard Instrument Company) was added to each of the vials to be mixed with the sample. Radioactivity contained per unit weight of each of the organs was measured by using a liquid sintillation counter (2500TR; product of Packard Instrument Company) to calculate an intra-tissue concentration of each of the siRNA administered as the complex.

(4) Conclusion

As is apparent from Fig. 14, the complex having the average particle diameter of 43.3 nm showed a remarkably reduced uptake by the reticuloendothelial system such as the liver and the spleen, and a higher plasma concentration thereof as compared to the complex having the average particle diameter of 187.5 nm.

Test Example 9: Effect of antisense DNA using the resent liposome

In each of petri dishes having a diameter of 6 cm, $2.5 \times 10^5$ cells of A431 cells were seeded and cultured overnight. On the following day, the culture media were replaced with fresh culture media (2.25 mL), and then 0.25 mL of the complex solution prepared by the method described in Example 3 and containing the antisense DNA (SEQ ID No. 5) for bcl-2 was added to the culture media. A concentration of the complex solution was adjusted to 10 times that of an final concentration using 10% maltose solution to be added to the culture media. Meanwhile, a complex containing the reverse sequence (SEQ ID No. 6) was prepared, and was added simultaneously. The cells were collected 72 hours after the addition, and expression of the Bcl-2 protein was analyzed in the same manner as in the method described in Test Example 1.

As shown in Fig. 15, it was possible to specifically suppress the expression of Bcl-2 protein by introducing the antisense DNA for the bcl-2 into a cell by the use of the present liposome.

Test Example 10: Effect of DNA enzyme using the present liposome

In each of petri dishes having a diameter of 6 cm, $2.5 \times 10^5$ cells of A431 cells were seeded and cultured overnight. On the following day, the culture media were replaced with fresh culture media (1.8 mL), and then 0.2 mL of a complex solution prepared by the method described in Example 4 and containing the DNA enzyme (SEQ ID No. 7) for Bcl-2 was added to the culture media to adjust the final concentration to 1 μM. The concentration of the complex solution was adjusted to 10 times that of the final concentration using 10 % maltose solution to be added to the culture media. Meanwhile, a complex containing the DNA enzyme (SEQ ID No. 8) into which a point mutation is so introduced as to lose its enzyme activity was prepared as a negative control, and was added simultaneously. The culture media were replaced with fresh culture media again 24 hours after the complex addition, and then the same complex was added to the culture media. The cells were collected 72 hours after the first complex addition, and expression of the Bcl-2 protein was analyzed in the same manner as in the method described in Test Example 1.

As shown in Fig. 16, it was possible to specifically suppress the expression of Bcl-2 protein by introducing the DNA enzyme for Bcl-2 into a cell by the use of the present liposome.

Industrial Applicability

The present invention provides a complex of a cationic liposome with an oligo nucleic acid, wherein the liposome comprises 2-O-(2-diethylaminoethyl)carbamoyl-1,3-O-dioleoylglycerol and a phospholipid as main ingredients. It is possible to administer the complex in vivo, and it is possible to practically use the complex as a medicament because the complex can exhibit pharmacological efficacy in vivo. It is possible to use the complex as a pharmaceutical composition for treating or preventing diseases caused by a target molecule of the oligo nucleic acid (target DNA, target RNA, target protein).

## Claims

1. A complex of a cationic liposome with an oligo nucleic acid having 10 to 50 nucleic acid bases, wherein the liposome comprises 2-O-(2-diethylaminoethyl)carbamoyl-1,3-O-dioleoylglycerol and a phospholipid as main ingredients.

2. The complex according to claim 1, wherein the phospholipid is lecithin.

3. The complex according to claim 1 or 2, wherein an average particle diameter is in the range of 10 to 100 nm.

4. The complex according to any one of claims 1 to 3, wherein the oligo nucleic acid is an RNA, a DNA, or a derivative of the RNA or the DNA.

5. The complex according to claim 4, wherein the RNA, the DNA, the derivative of the RNA or the DNA is an siRNA, an shRNA, an antisense DNA, an antisense RNA, a DNA enzyme, a ribozyme, or an aptamer.

6. The complex according to any one of claims 1 to 4, wherein the oligo nucleic acid is an oligo double-stranded RNA which comprises a double-strand forming part consisting of a sense strand RNA of the SEQ ID No. 1 from which 2 bases of dT at 3' end are eliminated, and an antisense strand RNA of the SEQ ID No. 2 from which 2 bases of dT at 3' end are eliminated.

7. The complex according to any one of claims 1 to 6, which is delivered to a cell in which a target molecule of the oligo nucleic acid (target DNA, target RNA, target protein) is present.

8. The complex according to claim 7, wherein the target molecule of the oligo nucleic acid is a oncogene such as a bcl-2, a c-myc, and a bcr-abl; a viral gene such as an HIV virus, a hepatitis C virus, and a hepatitis B virus; an inflammation-related gene such as TNF-$\alpha$ and Fas; a transcription product of any one of the genes; a translation product of any one of the genes; a genome DNA which does not encode any proteins; or an RNA acting for regulating protein expression.

9. A pharmaceutical composition for treating or preventing a disease caused by the target molecule of the oligo nucleic acid (target DNA, target RNA, target protein), comprising the complex according to any one of claims 1 to 8.

10. The pharmaceutical composition according to claim 9, wherein the disease is a cancer.

11. The pharmaceutical composition according to claim 9, wherein the disease is a viral disease.

**12.** The pharmaceutical composition according to claim 9, wherein the disease is an inflammatory disease.

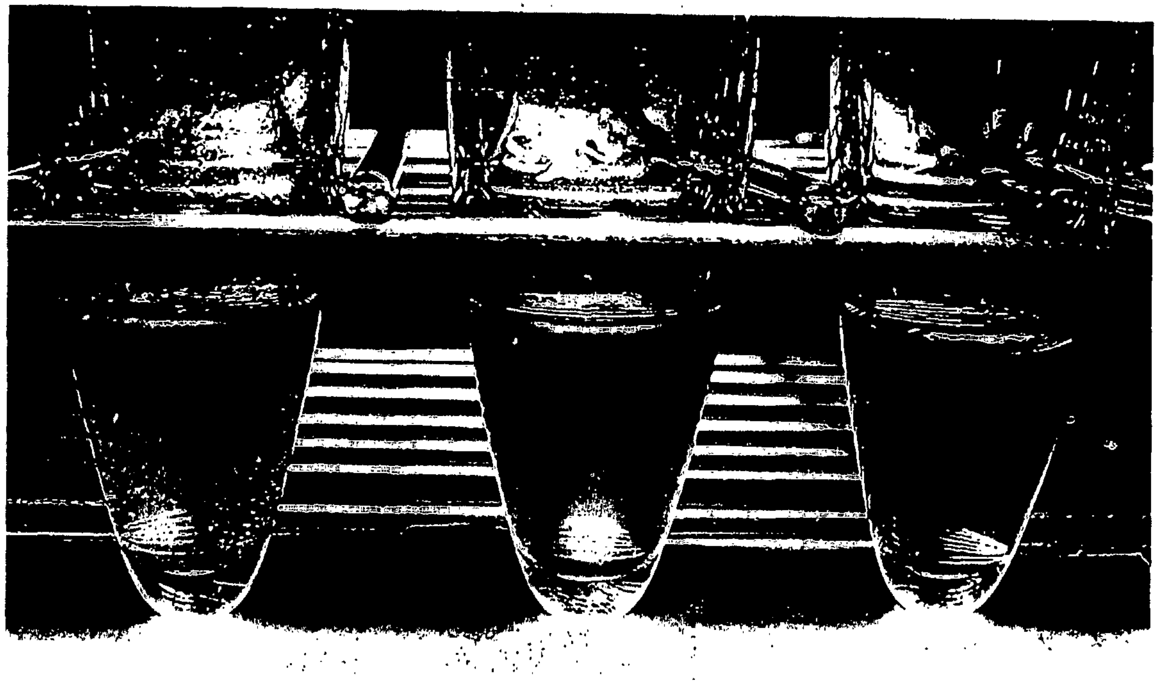

10 μM　　3 μM　　1 μM

Figure 1 : siRNA/Oligofectamine complex

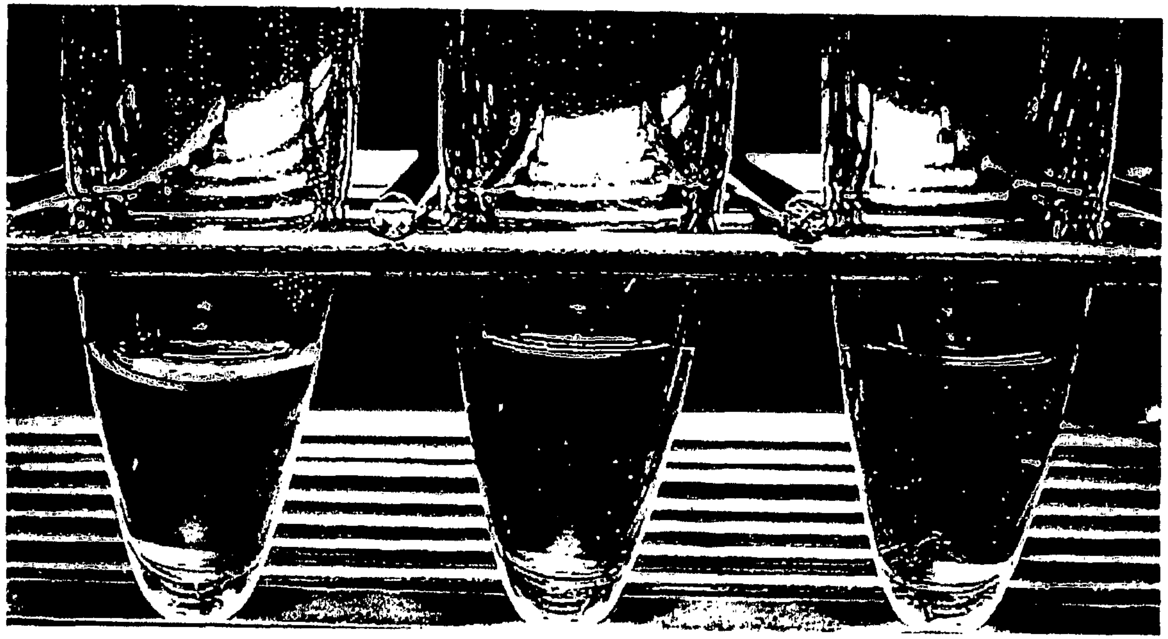

10 μM 3 μM 1 μM

Figure 2 : siRNA/CLZ42 liposome complex

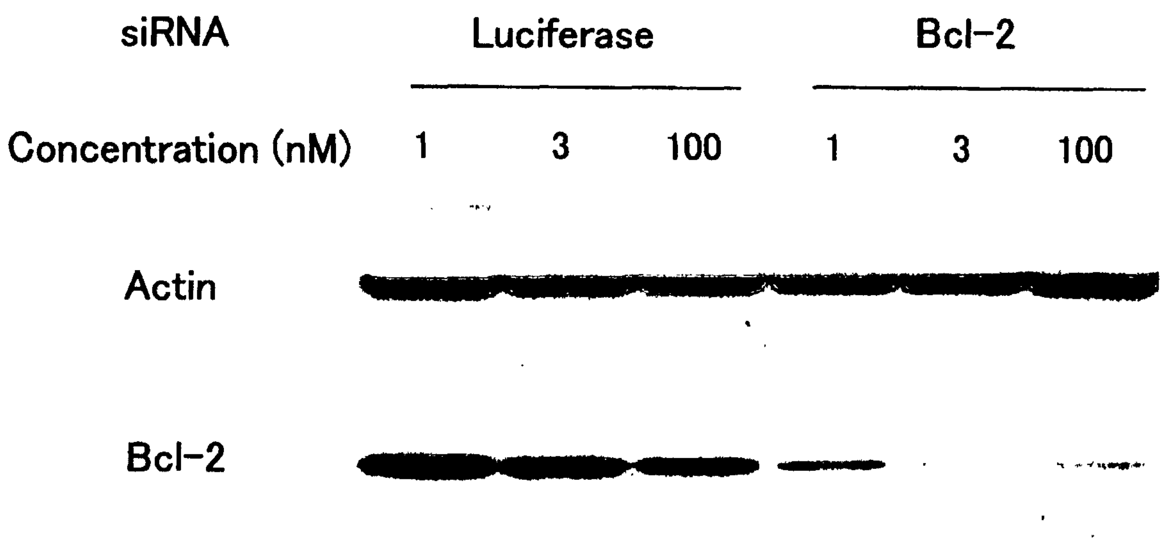


Figure 3 : Inhibition of Bcl-2 protein expression by siRNA in A431 cells

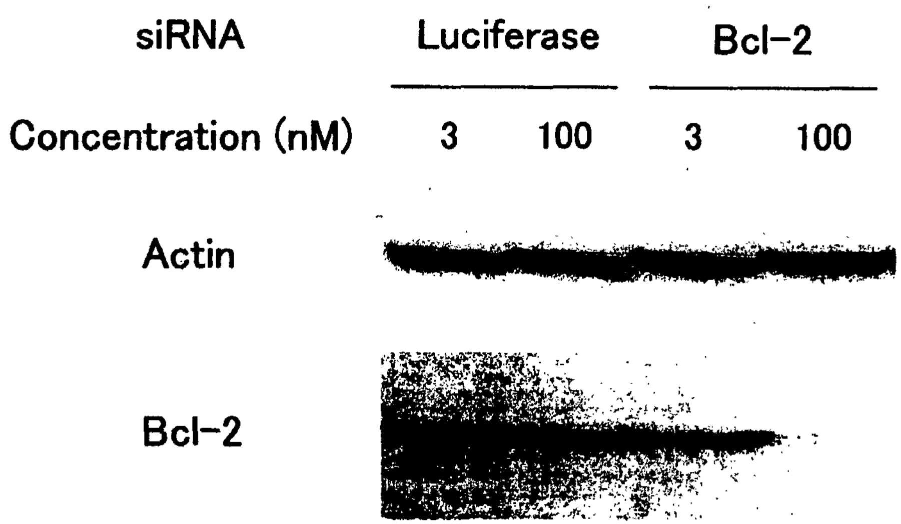


Figure 4 : Inhibition of Bcl−2 protein expression by siRNA in A549 cells

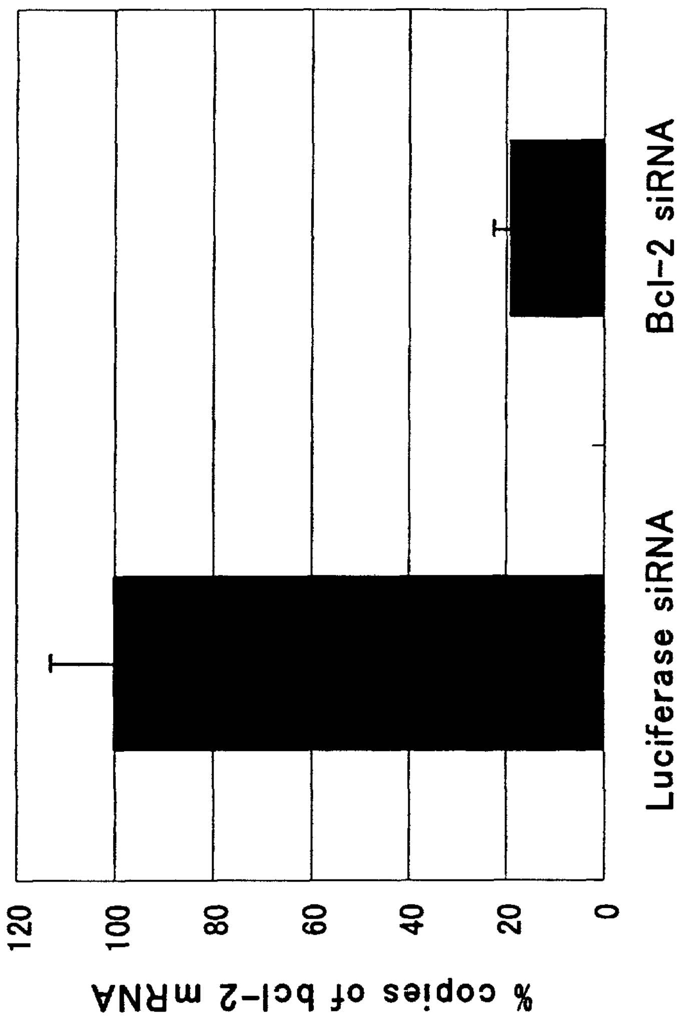


Figure 5 : Inhibition of bcl-2 mRNA expression by siRNA

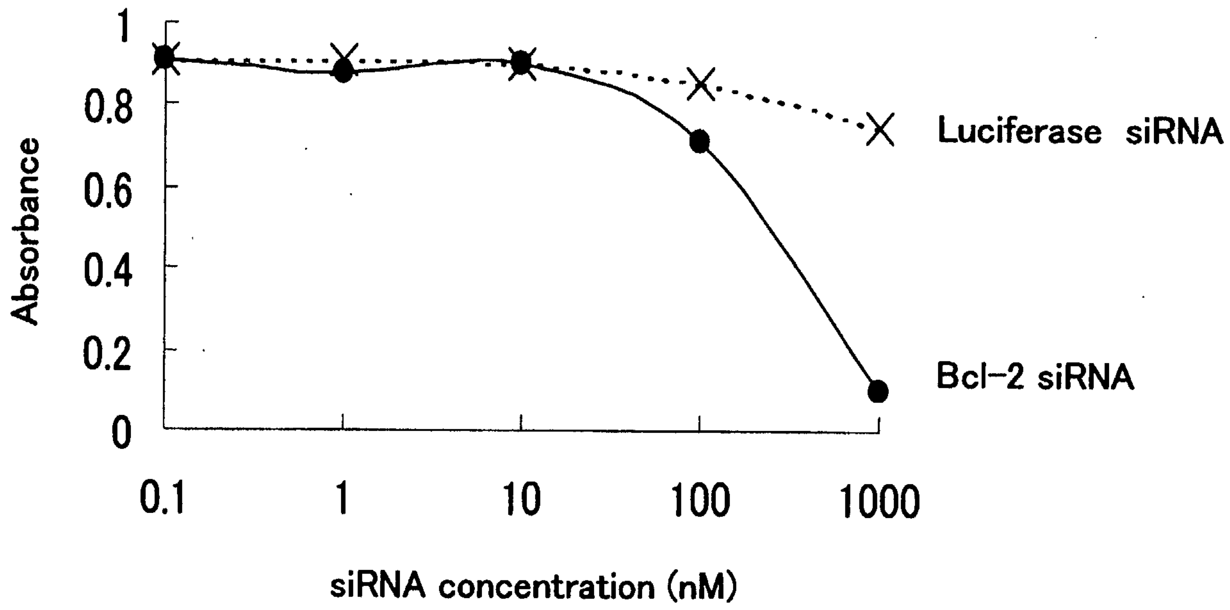


Figure 6 : Activity of cell growth inhibition by siRNA in A549 cells

siRNA complex

Naked siRNA

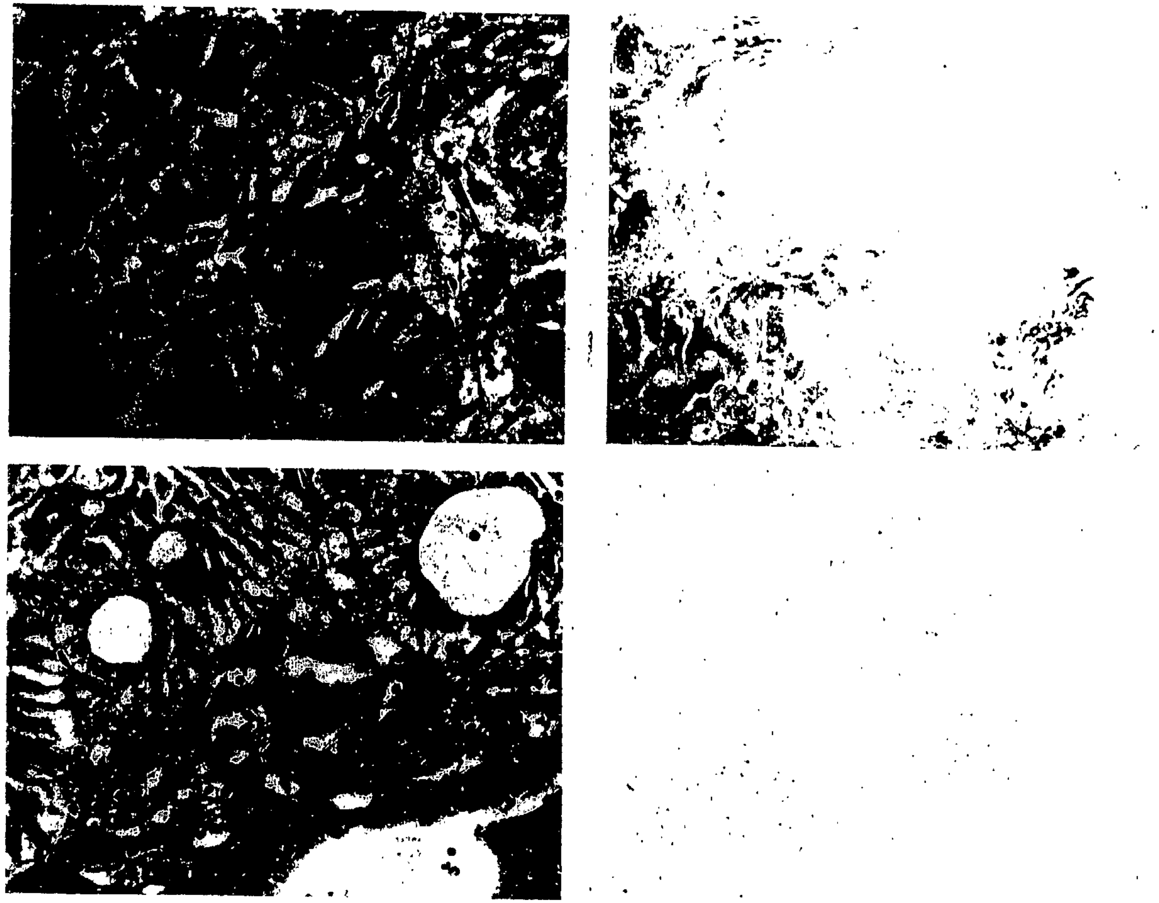

hematoxylin and eosin staining

immunohistochemical staining
(staining with alkaline phosphatase)

Figure 7 : Localization of siRNA complex in foci of metastasized liver tumor

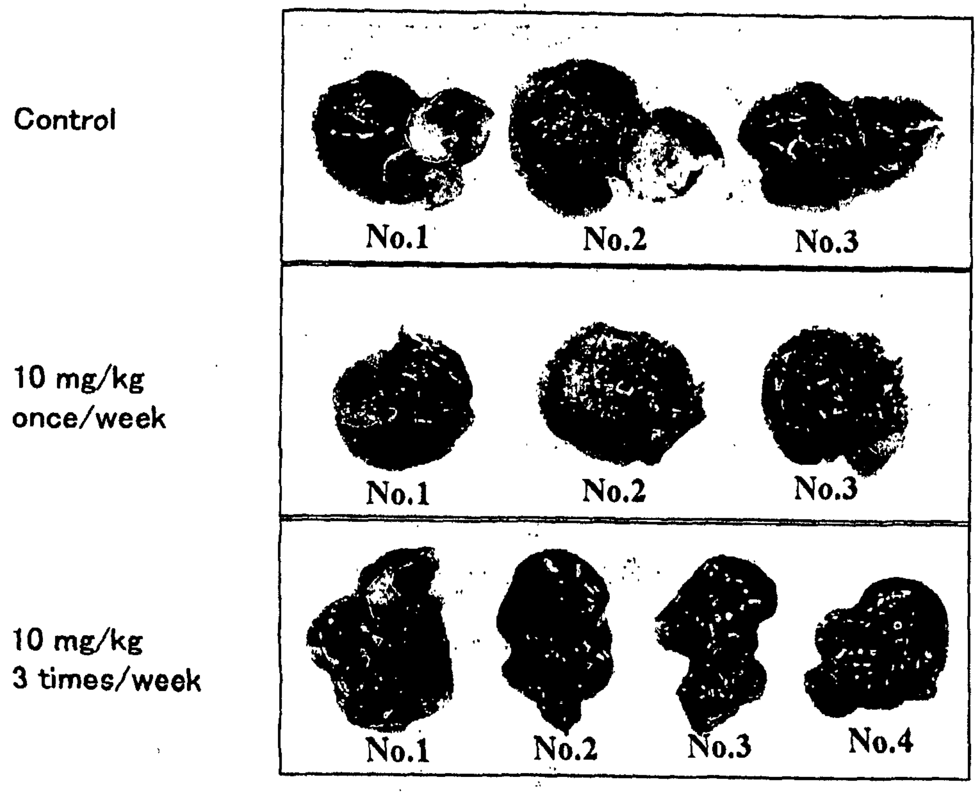


Figure 8 : Antitumor activity of siRNA complex in a mouse model of liver metastasis (inhibition of increase of tumor weight)

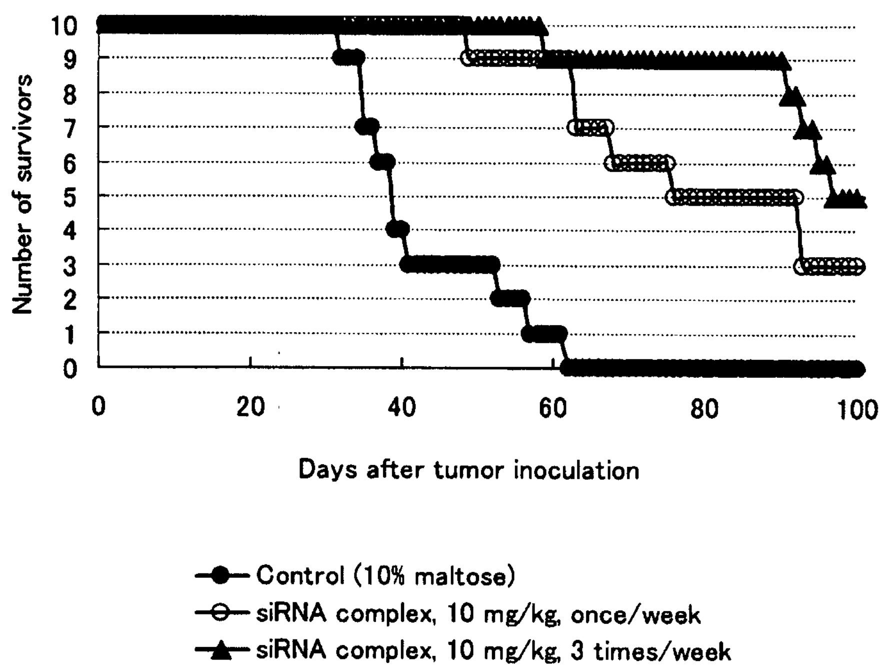


Figure 9 : Antitumor activity of siRNA complex in a mouse model of liver matastasis (extension of survival period)

# Figure 10

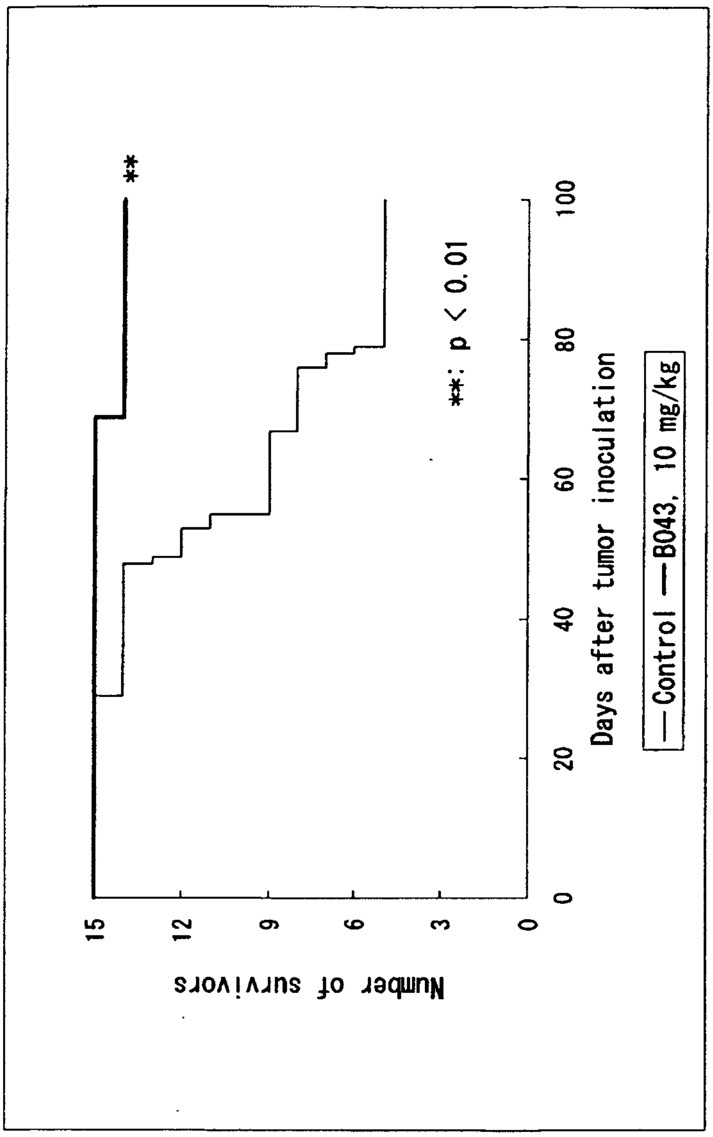

**
**: p < 0.01
Number of survivors
15
12
9
6
3
0
0
20
40
60
80
100
Days after tumor inoculation
Control
B043, 10 mg/kg

# Figure 11

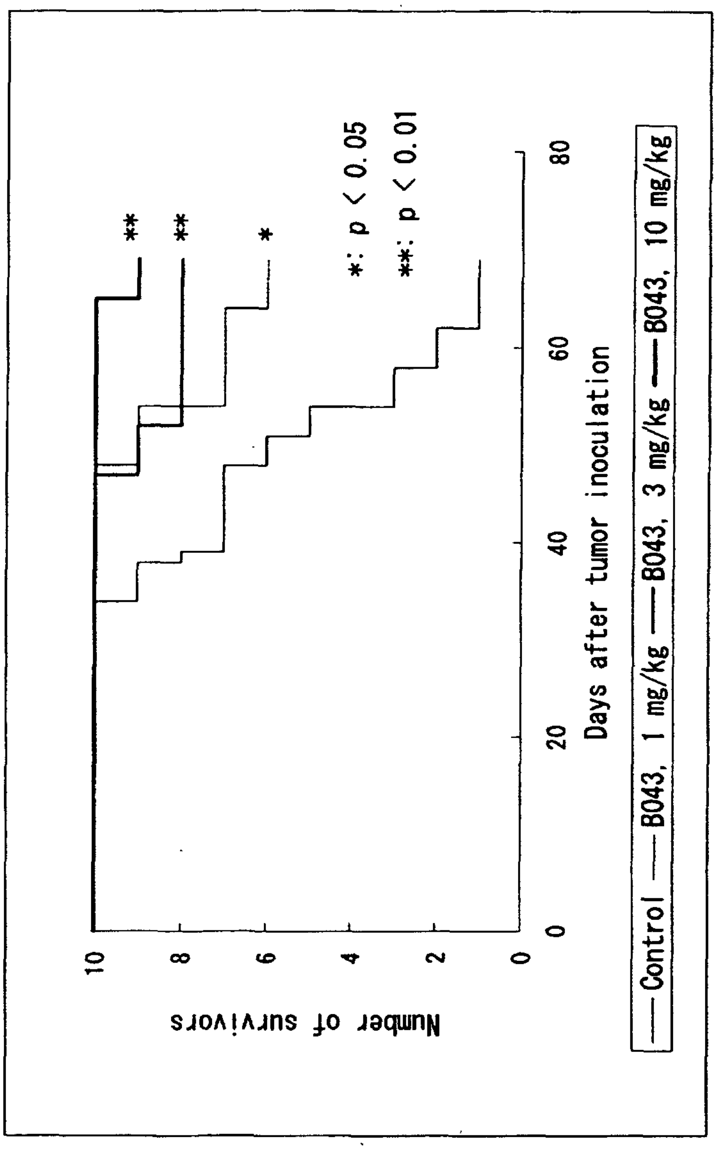
Number of survivors
10
8
6
4
2
0
0
20
40
60
80
Days after tumor inoculation
**
**
*
*: p < 0.05
**: p < 0.01
Control
B043, 1 mg/kg
B043, 3 mg/kg
B043, 10 mg/kg

# Figure 12

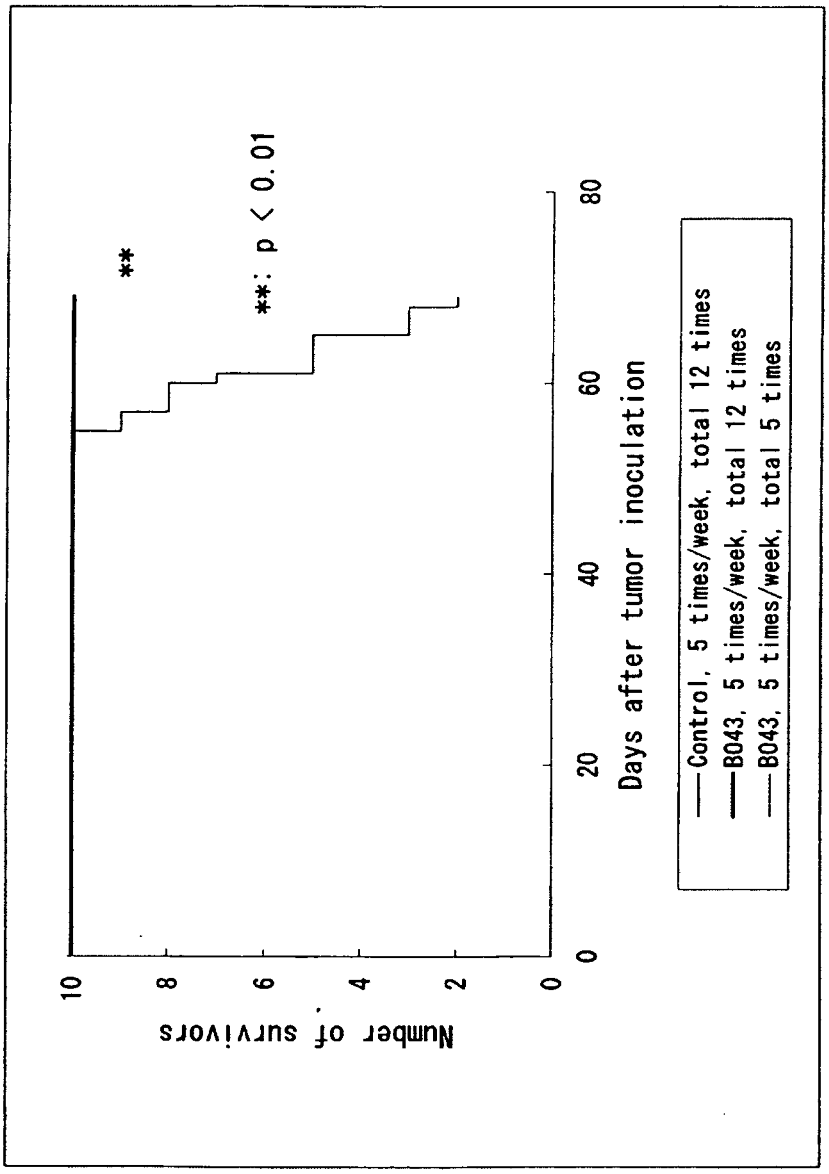
Number of survivors
0
2
4
6
8
10
0
20
40
60
80
Days after tumor inoculation
**
**: p < 0.01
Control, 5 times/week, total 12 times
B043, 5 times/week, total 12 times
B043, 5 times/week, total 5 times

# Figure 13

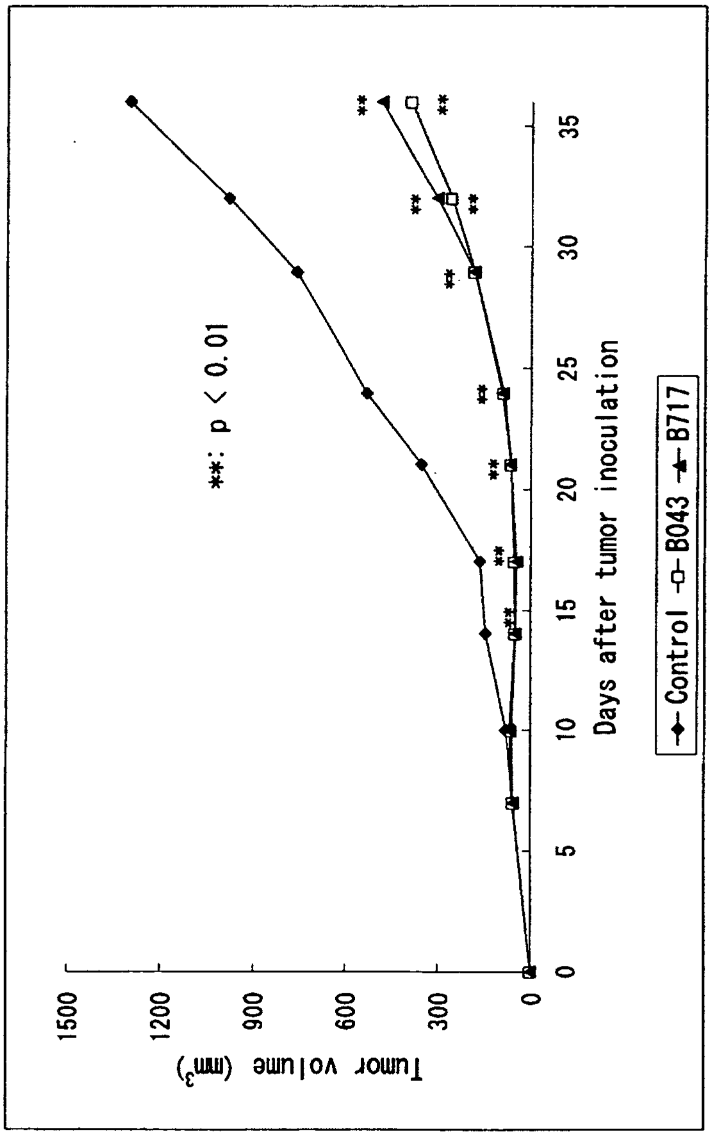
**: p < 0.01
Tumor volume (mm³)
1500
1200
900
600
300
0
0
5
10
15
20
25
30
35
Days after tumor inoculation
Control
B043
B717

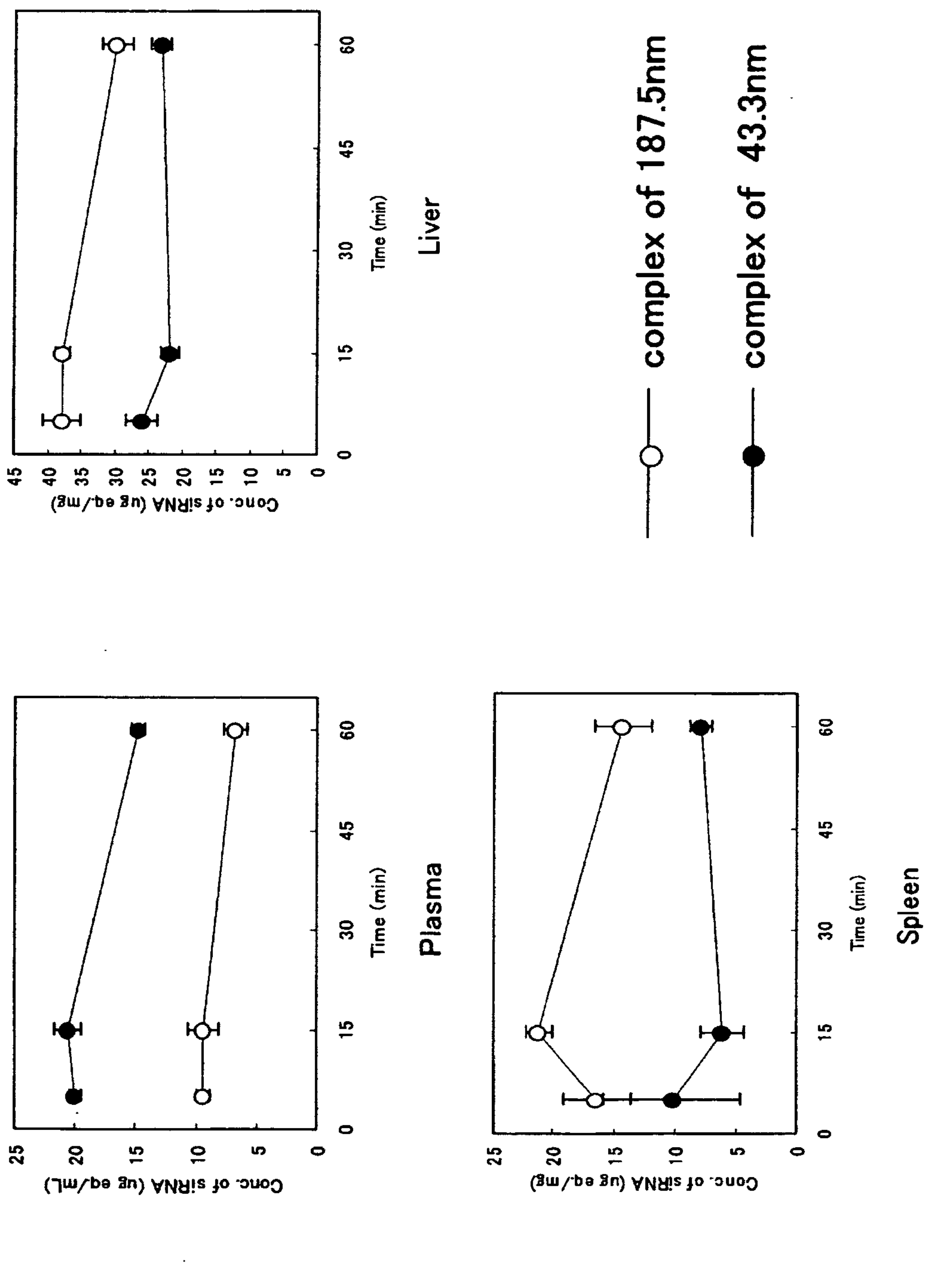


Figure 14 : Comparison of intratissue concentration of siRNA

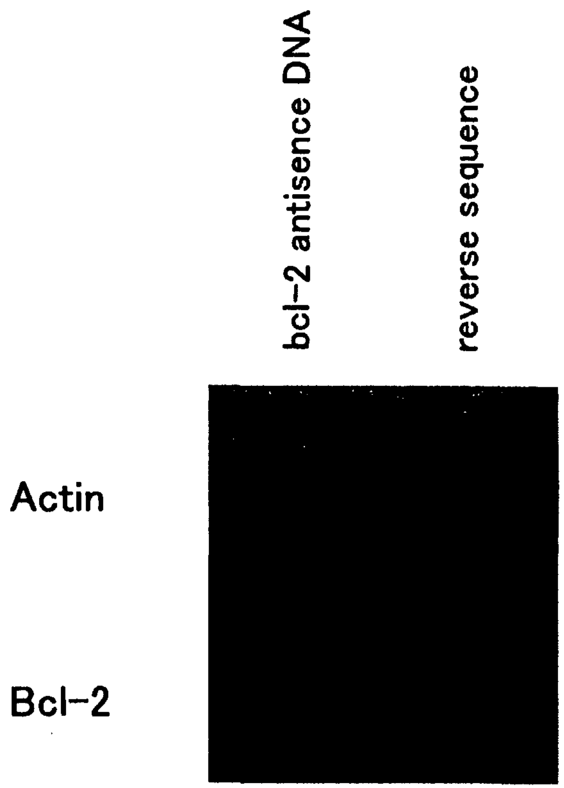


Figure 15 : Inhibition of Bcl-2 protein expression by antisence DNA

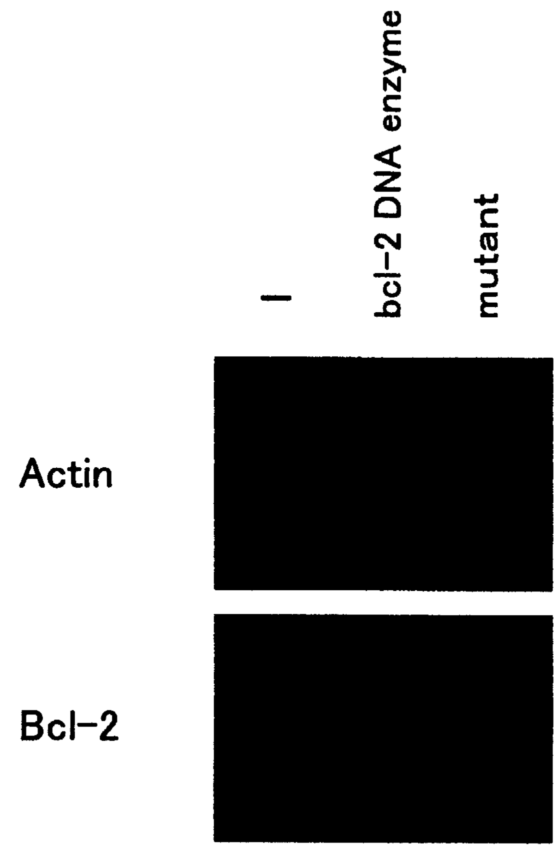


Figure 16 : Inhibition of Bcl-2 protein expression by DNA enzyme

**INTERNATIONAL SEARCH REPORT**

International application No.
PCT/JP2004/007785

A. CLASSIFICATION OF SUBJECT MATTER
Int.Cl$^7$ A61K31/7088, 9/127, 47/18, 47/24

According to International Patent Classification (IPC) or to both national classification and IPC

B. FIELDS SEARCHED

Minimum documentation searched (classification system followed by classification symbols)
Int.Cl$^7$ A61K31/7088, 9/127, 47/18, 47/24

Documentation searched other than minimum documentation to the extent that such documents are included in the fields searched

Electronic data base consulted during the international search (name of data base and, where practicable, search terms used)
CA(STN), REGISTRY(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| Y | WO 98/51278 A2 (Inex Pharmaceuticals Corp.), 19 November, 1998 (19.11.98), Full text & AU 733310 B & EP 1027033 A2 & JP 2002-501511 A | 1-12 |
| Y | WO 94/19314 A (Nippon Shinyaku Co., Ltd.), 01 September, 1994 (01.09.94), Full text & AU 9460449 A & JP 06-518814 A & EP 685457 B1 & US 6020317 A | 1-12 |
| Y | WO 99/20283 A (Nippon Shinyaku Co., Ltd.), 29 April, 1999 (29.04.99), Full text & AU 743137 B & EP 1029544 A | 1-12 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 August, 2004 (23.08.04) | 07 September, 2004 (07.09.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/007785

C (Continuation). DOCUMENTS CONSIDERED TO BE RELEVANT

| Category* | Citation of document, with indication, where appropriate, of the relevant passages | Relevant to claim No. |
|---|---|---|
| Y | Annemarie Ziegler et al., Induction of Apoptosis in Small-Cell Lung Cancer Cells by and Antisense Oligodeoxynucleotide Targeting the Boc-2 Coding Sequence, Journal of the National Cancer Institute, Vol.89, No.14, 1997, pages 1027 to 1036 | 6 |
| Y | WO 98/56905 A (NOVARTIS AG.), 17 December, 1998 (17.12.98), Full text & AU 9879183 A & US 6291668 B1 | 6 |
| Y | Dag R. Sorensen et al., Gene Silencing by Systemic Delivery of Synthetic siRNAs in Adult Mice, J.Mol.Biol. Vol.327, pages 761 to 766, April 2003 | 1-12 |

Form PCT/ISA/210 (continuation of second sheet) (January 2004)